# EUROPEAN PATENT APPLICATION

(11) **EP 0 865 823 A1**
(43) Date of publication of application: **23.09.1998**
(21) Application number: 98302068.6
(22) Date of filing: 19.03.1998
(51) Int. Cl.: B01J 29/04, B01J 21/08, C07C 2/68

(54) **Catalyst based on aluminium halide and its use in Friedel-Crafts alkylation of benzene using long chain olefins**

(30) Priority: 20.03.1997 GB 9705799
(71) Applicant: CONTRACT CHEMICALS LIMITED, Prescot, Merseyside L34 9HY (GB)
(72) Inventor: Trenbirth, Brian, Haslington Crewe (GB); Bastock, Tony William, Sandbach Cheshire (GB); Clark, James Hanley, York (GB); Martin, Keith, West Derby Liverpool (GB); Price, Peter, Walton Chesterfield (GB)
(74) Representative: W.P. Thompson & Co.

(57) **Abstract**

There is disclosed a catalyst, particularly a Friedel-Crafts alkylation catalyst, which comprises an aluminium halide of the formula AlXₙR₃₋ₙ, where X is halogen preferably Cl, R is a substituted or unsubstituted alkyl, preferably methyl or ethyl, or a substituted or unsubstituted aryl, preferably phenyl and n is 1, 2 or 3, (preferably 2 or 3), chemically bonded to Hexagonal Mesoporous Silica (HMS) or derivitised HMS. Also disclosed are methods for the production of the catalyst and its use in alkylation reactions.

## Description

The present invention relates to a catalyst and to the use of a catalyst in Friedel-Crafts alkylation of benzene using long chain olefins.

There are a variety of catalysts known for the alkylation of benzene with long-chain olefins. However, there are many disadvantages associated with such catalysts, these include the corrosive nature of the catalyst; difficulties in separation and recovery causing large volumes of environmentally hazardous waste; and low selectivity towards the desired product. The use of solid acid catalysts eliminate these problems, however, they do present a set of their own problems, such as the need for high temperatures and pressures, along with low conversion and coking problems.

At present there are three main catalysts used for the alkylation of benzene with long-chain olefins, aluminium tri-chloride, hydrogen fluoride and zeolites, although there are several others which may be employed.

Aluminium tri-chloride is the most widely used Friedel-Crafts alkylation catalyst and so has been studied extensively, and several reviews on the reactions are available, these include "Friedel-Crafts and related reactions", Olah, Wiley, New York, 1963-1965, "Advanced organic chemistry", March, Wiley interscience, New York, p534-539.

Both aluminium tri-chloride and hydrogen fluoride are used as catalysts in industry for the alkylation of benzene using long-chain olefins for the production of Linear Alkylbenzenes (LAB), with the process involved appearing in several reviews by J.L.G. de Almeida et al J.A.O.C.S., vol 71, No. 7, July 1994, 675-694; J.L. Berna, L.Cavalli, C. Renta, Tenside Surf. Det. 32,2,1995, 122-127.

There has been recent interest in the use of solid acid catalysts (namely zeolites) for the use in the alkylation of benzene with long chain olefins publications include S. Sivasanker, A. Thangaraj, Journal of Catal., 138, 386-390 (1992); W. Liang et al, Zeolites, 17, 297-303, 1996.

Both aluminium tri-chloride and hydrogen fluoride show good activity but poor selectivity, whereas present solid acid catalysts show good selectivity but poor activity. It is therefore important that the catalyst shows both good activity and selectivity and should be heterogeneous to reduce environmental impact. Such catalysts can be based on Lewis acids bound to inorganic supports such as described in R.S. Drago, E.E. Getty, J.A.C.S., 1988, 110, 3311-3312; E.E. Getty, R.S. Drago, Inorg. Chem., 1990, 1186-1192; J.H. Clark, K. Martin, A.J. Teasdale, S.J. Barlow, Chem. Commun. 1996, 2037-2040.

According to the present invention there is provided a catalyst, particularly a Friedel-Crafts alkylation catalyst, which comprises an aluminium halide of the formula A1XₙR₃₋ₙ, where X is halogen preferably Cl, R is a substituted or unsubstituted alkyl, preferably methyl or ethyl, or a substituted or unsubstituted aryl, preferably phenyl and n is 1, 2 or 3, (preferably 2 or 3), chemically bonded to Hexagonal Mesoporous Silica (HMS) or derivitised HMS, the preparation of which is achieved by known methods.

The preferred aluminium halide is aluminium trichloride.

The catalyst may be prepared by known methods.

The present invention also provides a method of preparing a catalyst of the invention by chemically binding an aluminium halide as defined above to HMS or derivitised HMS.

One method of preparing the catalyst comprises of slurrying an aluminium halide, in a solvent at room temperature, adding a pre-dried HMS or derivitised HMS support material, warming the mixture to reflux and cooling, to give a slurry of the catalyst in the solvent which may be used directly or filtered, washed and dried, preferably under an inert atmosphere for example nitrogen to yield the dry powdered catalyst. The dried catalyst should then be stored in dry, inert conditions to minimise loss of activity.

The solvent should be inert with regard to the materials used and is preferably weakly complexing with regard to aluminium. Preferred solvents are hydrocarbons and halohydrocarbons.

The concentration of the slurry used is not of importance, bearing in mind the amount of solid and size of any vessel in which the catalyst is being prepared, together with any limitation on the agitation possible.

The solvents usually employed are selected from hydrocarbons and halohydrocarbons, particularly useful are aromatic hydrocarbons such as benzene or substituted benzenes. It is not necessary for the aluminium halide to be soluble in the solvent to any appreciable extent but it can be advantageous.

The essential feature of the invention relates to the attachment of the aluminium halide to the internal surface (i.e. within the pores) of the HMS material. This is achieved by reacting the aluminium halide with the internal hydroxyl groups of the HMS giving a heterogeneous catalyst not prone to leaching.

It is also preferred to remove external sites either by removing the external hydroxyls of the HMS by reacting the hydroxyl groups with a bulky silane group eg triphenylsilane prior to the supporting of aluminium tri-chloride or, by poisoning any external catalytic sites with a bulky amine eg triphenylamine. This results in further selectivity benefit by forcing more of the chemistry to occur in this internal structure.

One method of preparing the Freidel-Crafts alkylation catalyst involves adding the aluminium halide to the HMS or derivitised HMS and refluxing together in a suitable solvent for 1 hour. The loading of the aluminium halide is typically in the range of 0.01 to 10 preferably 0.75 to 3.0, and most preferably 0.75 to 1.5 mmol per gram of HMS or derivitised HMS. The catalyst may then be used in-situ or separated from the solvent under vacuum in an atmosphere of nitrogen.

According to another embodiment of the present invention there is provided a process for the alkylation of an organic substrate (eg a cyclic organic substrate eg benzene or a substituted benzene which comprises reacting the organic substrate with an alkylating agent (eg a long chain olefin, preferably a linear olefin) in the presence of a catalyst in accordance with the invention.

The catalyst may be prepared in-situ in benzene or in another suitable solvent or may have been prepared and isolated and then added to benzene or a suitable solvent when about to be used. The alkylating agent (eg long chain olefin) may then be added to the mixture at a desired rate and the reaction continued for the appropriate time. The catalyst may be separated from the reaction by filtration, decantation or a similar technique under an inert atmosphere. In contrast with many known processes the catalyst may be reused.

The product of the alkylation can then be isolated and purified by standard, known techniques.

Examples of organic substrates are open chain compounds or cyclic compounds and may be substituted or unsubstituted.

Suitable cyclic organic substrates which may be used in the process of the present invention include those compounds having the general formula: - 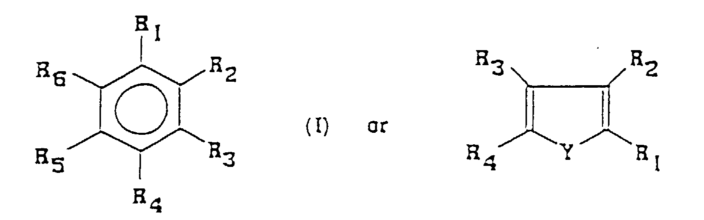 wherein R₁ to R₆ may independently each be: -
(a) H, D (deuterium), F, Cl, Br, I;
(b) alkyl;
(c) OR;
(d) SR;
(e) NR₇R₈;
(f) COR;
(g) COOR;
(h) R₉-C=C-R₁₀R₁₁;
(i) C≡C-R
(j) CN,CHO;
(k) NO,NO₂
wherein R and R7 to R11 may independently each be H,D, alkyl, aryl or acyl which may be substituted; and Y may be O, NH, ND, NR (wherein R is as defined above), or S.

Any two substituents may be joined to form a ring, which in turn may be substituted.

Suitable alkylating agents which may be used in the process of the present invention include: -
(1) R1R2R3CX
(2) R1R2C=CR3R4
(3) R1C≡CR2
(4) R1R2
wherein R1 to R4 are independently each as defined hereinabove for the organic substrates, and X is F, Cl, Br, I, o-tosyl, OH or OR (R being as hereinabove defined).

The substance to be alkylated (the substrate) is reacted with the alkylating agent in the presence of the catalyst. The molar ratio of reactants is usually (substrate: agent) in the range 1 to 20, preferably 2 to 10. However, it may be less than 1 for some systems.

The amount of catalyst employed can range widely depending on the reactants and whilst it may be within the wide range of 0.1 to 100% it is usually 0.1 to 10% for alkylation reactions. The percentages are on a weight basis and are based on the weight of alkylating agent.

The temperature at which the alkylation is conducted usually depends on the particular reaction, but is generally in the range -100°C to 300°C. Preferably the alkylation is carried out at room temperature (ie approx 15 to 25°C).

Whilst the alkylating agent may be added slowly to the reaction medium, it may be added totally at the commencement. It may be that the catalyst forms part of a "bed" fixed or otherwise over which the mixed reactants are passed in a continuous system. The reaction medium may also contain a solvent which may be a saturated or aromatic hydrocarbon of lower activity than the substrate, or a chlorinated hydrocarbon such as, for example chloroform.

It is also possible to conduct any process under ambient or elevated pressures, especially of any of the components are particularly volatile at the temperature of the reaction.

On completion of the reaction the catalyst may be removed from the reaction medium by filtration or decantation and then re-used. Purification and isolation of the required product may be performed by conventional means.

The capability of recovering and reusing the supported reagent catalyst is an important advantage over much of the current alkylation technology.

The present invention will now be further described with reference to, but is in no manner limited to, the following examples.

### Example 1

A catalyst was prepared by adding anhydrous aluminium tri-chloride (0.40g, 3mmol) to dry Hexagonal Mesoporous Silica, with a 24A pore size, (2g) in sodium dried benzene (20ml) which was refluxed under a nitrogen atmosphere for lh. The mixture was cooled to room temperature and was used in-situ for the alkylation of benzene.

### Example 2

To the catalyst of Example 1 (0.40g of aluminium tri-chloride supported on HMS, 24A, prepared in benzene) without isolation from the benzene solvent was added triphenylamine (0.147g, 0.5mmol) dissolved in sodium dried benzene (1ml). The catalyst and the triphenylamine were stirred together under a nitrogen atmosphere for 30 min. The catalyst was then used in-situ for the alkylation of benzene.

### Example 3

A derivitised HMS is prepared by treating pre-dried (300C for 18h) HMS (2g) with triphenylsilychloride (TPSC1) (17.69g, 0.06mol) dissolved in sodium dried toluene (39ml). This was then refluxed under a nitrogen atmosphere for 18h. The derivitised HMS was isolated by filtration, washed with acetone and dried by vacuum. The oven dried (200°C for 18h) derivitised HMS (HMS-TPS) was then used to prepare a catalyst in accordance with the invention by a method as described in Example 1 but using 0.20g, aluminium tri-chloride and 2g, HMS-TPS prepared in benzene.

### Example 4

To the catalyst of Example 1 0.40g, aluminium tri-chloride on 2g, HMS 24A prepared in-situ in benzene (20ml, 0.2mol) without isolation from the benzene solvent was added 1-dodecene (22.2ml, 0.1mol) dropwise over a period of 30-60 min, via a peristaltic pump. The reaction mixture was placed in a water bath to keep at a constant temperature of about 15 to 25°C (ie room temperature), with nitrogen flowing through the system. Analysis of the reaction mixture by GC showed 100% conversion after the final addition of 1-dodecene (30min), with a selectivity towards mono-dodecylbenzene of 81% (42% of which was the 2-phenyl isomer).

### Example 5

To the catalyst of Example 2 (0.40g, aluminium tri-chloride on 2g, HMS, 24A prepared in-situ in benzene 20ml, 0.2mol without isolation from the benzene solvent) was added 0.5mmol of triphenylamine. To this mixture was added 1-dodecene (22.2ml, 0.1mol) dropwise over a period of 30min via a peristaltic pump. The reaction mixture was placed in a water bath to keep at constant temperature of about 15 to 25°C (ie room temperature), with nitrogen flowing through the system. Analysis of the reaction mixture by GC showed 100% conversion after 30min, with a selectivity towards mono-dodecylbenzene of 86% (39.1% of which was the 2-phenyl isomer).

### Example 6

To the catalyst of Example 3 (0.20g aluminium tri-chloride on 2g, HMS-TPS, 24A prepared in-situ in benzene 20ml, 0.2mol), without isolation from the benzene solvent was added 1-dodecene (22.2ml, 0.1mol) dropwise over a period of 30min. The reaction was placed in a water bath to keep at a constant temperature of about 15 to 25°C (ie room temperature), with nitrogen flowing through the system. Analysis of the reaction mixture by GC showed 100% conversion after 30min, with a selectivity towards mono-dodecylbenzene of 84.5% (41.2% of which was the 2-phenyl isomer).

### Example 7

To the catalyst of Example 1 (0.40g, aluminium tri-chloride on 2g, HMS 24A prepared in-situ in benzene 20ml, 0.2mol), without isolation from the benzene solvent was added 1-octene (16ml, 0.1mol) dropwise over a period of 30 min, via a peristaltic pump. The reaction mixture was placed in a water bath to keep at constant temperature of about 15 to 25°C (ie room temperature), with nitrogen flowing through the system. Analysis of the reaction mixture by GC showed 100% conversion after 30 min, with a selectivity towards mono-octylbenzene of 80%.

### Example 8

To the catalyst of Example 1 (0.40g, aluminium tri-chloride on 2g, HMS 24A prepared in-situ in benzene 20ml, 0.2mol) without isolation of the benzene solvent was added 1-tetradecene (25.3ml, 0.1mol) dropwise over a period of 30min, via a peristaltic pump. The reaction was placed in a water bath to keep at a constant temperature of about 15 to 25°C (ie room temperature), with nitrogen flowing through the system. Analysis of the reaction mixture by GC showed 100% conversion after 30min, with a selectivity towards mono-tetradecylbenzene of 86.9% (38.5 % of which is the 2-phenyl isomer).

### Example 9

The catalyst of example 1 (0.4g, aluminium tri-chloride on 2g, HMS 24Å prepared in-situ in benzene 20ml, 0.2mol) was isolated and added to ethylbenzene (24.5ml, 0.2mol). To the slurry was added 1-octene (15.6ml, 0.1mol) dropwise over a period of 30 min, via a peristaltic pump. The reaction was placed in a water bath to keep at a constant temperature of about 15 to 25°C (ie room temperature), with nitrogen flowing through the system. Analysis of the reaction mixture by GC showed 100% conversion after 30min, with a selectively towards mono-octylethylbenzene of 85 % (60% of which is the para-isomer).

## Claims

1. A catalyst which comprises an aluminium halide of the formula AlXₙR₃₋ₙ, where X is halogen, R is a substituted or unsubstituted alkyl, or a substituted or unsubstituted aryl, and n is 1, 2 or 3, chemically bonded to Hexagonal Mesoporous Silica (HMS) or derivatised HMS.

2. A catalyst as claimed in claim 1, in which the aluminium halide is aluminium trichloride.

3. A catalyst as claimed in claim 1 or 2 in which the loading of aluminium halide is from 0.01 to 10 mmol per gram of HMS or derivatised HMS.

4. A catalyst as claimed in any one of the preceding claims, in which the loading of aluminium halide is from 0.75 to 3.0 mmol per gram of HMS or derivatised HMS.

5. A catalyst as claimed in any one of the preceding claims, in which the loading of aluminium halide is from 0.75 to 1.5 mmol per gram of HMS or derivatised HMS.

6. A method of preparing a catalyst of the invention comprising chemically binding an aluminium halide as defined above to HMS or derivatised HMS.

7. A method as claimed in claim 6 which comprises slurrying an aluminium halide, in a solvent at room temperature, adding a pre-dried HMS or derivitised HMS support material, warming the mixture to reflux and cooling, to give a slurry of the catalyst in the solvent which may be used directly or filtered, washed and dried to yield the dry powdered catalyst.

8. A process for the alkylation of an organic substrate which comprises reacting the organic substrate with an alkylating agent in the presence of a catalyst as claimed in any one of claims 1 to 5.

9. A process as claimed in claim 8, in which the catalyst is prepared in-situ.

10. A process as claimed in claim 8 or 9, in which the catalyst is present in an amount of 0.1 to 100% by weight based on the weight of the alkylating agent.

11. A process as claimed in claim 10, in which the catalyst is present in an amount of 0.1 to 10% by weight based on the weight of the alkylating agent.
